# EUROPEAN PATENT APPLICATION

(11) **EP 4 664 474 A1**
(43) Date of publication of application: **17.12.2025**
(21) Application number: 24182142.0
(22) Date of filing: 14.06.2024
(51) Int. Cl.: G16H 40/40, G16H 40/67, G16H 10/60, G16H 40/20

(54) **METHOD AND SYSTEM FOR MANAGING A MRI-CERTIFICATE OF AN ACTIVE IMPLANT SYSTEM**

(71) Applicant: BIOTRONIK SE & Co. KG, 12359 Berlin (DE)
(72) Inventor: DÖRR, Thomas, 12437 Berlin (DE)
(74) Representative: Biotronik Corporate Services SE

(57) **Abstract**

The invention relates to a computer-implemented method for performing an administrative action (18) concerning a MRI-certificate (10) of the active implant system (12), comprising the steps of providing (S1) the active implant system (12), said active implant system (12) comprising an implantable medical device (12a) and electrode leads (12b) connected to the implantable medical device (12a), transmitting (S2) technical and/or medical data (14) acquired by the active implant system (12), in particular via a relay device (28), to a remote monitoring system (16), based on the technical and/or medical data (14) acquired by the active implant system (12), verifying (S3) a MRI-compliance of the active implant system (12), and based on a result of the verification, performing an administrative action (18) concerning the MRI-certificate (10) of the active implant system (12). Furthermore, the invention relates to a system for performing an administrative action (18) concerning a MRI-certificate (10) of the active implant system (12).

## Description

The invention relates to a computer-implemented method for performing an administrative action concerning a MRI-certificate of an active implant system.

Furthermore, the invention relates to a system for performing an administrative action concerning a MRI-certificate of an active implant system.

Advances in magnetic resonance imaging and magnetic resonance angiography over the last two decades have led to magnetic resonance becoming an increasingly attractive imaging modality. Magnetic resonance imaging provides excellent spatial resolution and multiplanar 3-dimensional analysis, while not exposing patients to ionizing radiation or the risks of invasive procedures. In order to safely undergo magnetic resonance imaging, active implant systems are subject to MRI-certification.

For the currently required annual re-evaluation of the MRI suitability of active implants, the patient must present to the doctor, wherein the data for assessing the MRI suitability is requested with an in-office programming device and a certificate must be issued or renewed with said programming device.

The certificate is currently valid for one year, as the patient's conditions may change. In terms of the clinical workflow, an implant expert must confirm that the conditions of the implanted system correspond to what is described as MRI suitability, e.g. both the implant and the electrode must be MRI-compatible.

Moreover, these elements must be from the same manufacturer. No abandoned, switched-off electrodes may remain in the patient and certain electrical readings from the implant must be verified so that it can be confirmed that the patient can undergo an MRI. This MRI suitability verification process for implants however is time consuming for both the patient and the physician.

It is therefore an object of the present invention to provide a more efficient method for determining a MRI suitability for an active implant system.

The object is solved by a computer-implemented method for performing an administrative action concerning a MRI-certificate of the active implant system having the features of claim 1.

Furthermore, the object is solved by a system for performing an administrative action concerning a MRI-certificate of the active implant system having the features of claim 13.

Furthermore, the object is solved by a computer program having the features of claim 14 and by a computer-readable data carrier having the features of claim 15.

Further developments and advantageous embodiments are defined in the dependent claims.

The present invention provides a computer-implemented method for performing an administrative action concerning a MRI-certificate of the active implant system.

The method comprises providing the active implant system, said active implant system comprising an implantable medical device and electrode leads connected to the implantable medical device. Furthermore, the method comprises transmitting technical and/or medical data acquired by the active implant system, in particular via a relay device, to a remote monitoring system.

In addition, the method comprises, based on the technical and/or medical data acquired by the active implant system, verifying a MRI-compliance of the active implant system, and based on a result of the verification, performing an administrative action concerning the MRI-certificate of the active implant system.

The present invention further provides a system for performing an administrative action concerning a MRI-certificate of the active implant system.

The system comprises an active implant system, said active implant system comprising an implantable medical device and electrode leads connected to the implantable medical device, and a remote monitoring system configured to exchange data with the active implant system.

The active implant system is configured to transmit technical and/or medical data acquired by the active implant system, in particular via a relay device, to the remote monitoring system. Moreover, the remote monitoring system is configured to, based on the technical and/or medical data acquired by the active implant system, verify a MRI-compliance of the active implant system.

In addition, based on a result of the verification, the remote monitoring system is configured to perform an administrative action concerning the MRI-certificate of the active implant system.

Moreover, the present invention provides a computer program with program code to perform the method of the present invention when the computer program is executed on a computer. In addition, the present invention provides a computer-readable data carrier containing program code of a computer program for performing the method of the present invention when the computer program is executed on a computer.

An active implant system is defined as an implant system comprising a power source such as a pacemaker, a defibrillator, a heart rhythm monitor, a neurostimulator and/or a medication pump.

An idea of the present invention is to provide an improved method for certification and/or re-certification of the active implant system which can be carried out safely without the patient being present.

The present invention thus enables MRI certification to be carried out remotely without the patient having to be present at the doctor, i.e. the entire workflow can be significantly simplified. Furthermore, the invention achieves to reduce costs and increase acceptance by patients and practicing physicians.

The solution according to the invention hence significantly simplifies the workflow for certification as well as re-certification of MRI-capable implants.

According to an aspect of the invention, performing the administrative action concerning the MRI-certificate of the active implant system comprises issuing, revoking or re-issuing the MRI-certificate of the active implant system by the remote monitoring system.

The remote monitoring system is thus capable of performing any administrative action on an existing MRI-certificate or a MRI-certificate yet to be created.

According to a further aspect of the invention, the remote monitoring system updates an expiry date of the MRI-certificate in a data storage of the implantable medical device when the MRI-certificate is issued, revoked or re-issued.

The data storage of the implantable medical device thus advantageously holds up-to-date information on a current certificate status of a MRI capability of the implantable medical device.

According to a further aspect of the invention, the MRI-certificate has a specified period of validity, wherein the active implant system sends a first notification to the remote monitoring system to initiate re-issuing the MRI-certificate at a predefined point in time prior to expiration of the MRI-certificate.

Neither the patient wearing the implantable medical device nor the physician treating the patient thus needs to actively monitor a validity duration or expiry date of the MRI-certificate.

According to a further aspect of the invention, the active implant system transmits the technical and/or medical data, in particular via a relay device, to the remote monitoring system required for verifying the MRI-compliance of the active implant system at said predefined point in time prior to expiration of the MRI-certificate.

The remote monitoring system thus receives all relevant technical and/or medical data required for MRI-certification, revocation or re-certification of the implantable medical device.

According to a further aspect of the invention, the active implant system sends the technical and/or medical data required for verifying MRI-compliance of the active implant system with its daily data transmission to the remote monitoring system.

Active monitoring of an MRI-compliance status of the active implant system is thus advantageously measured on a daily basis.

According to a further aspect of the invention, the remote monitoring system evaluates all daily data transmissions and automatically issues or re-issues the MRI-certificate if the predefined MRI-requirements are met. This advantageously enables a seamless and efficient process for automatic verification, certification or re-certification of the MRI suitability of the implantable medical device.

According to a further aspect of the invention, the remote monitoring system evaluates all daily data transmissions and automatically revokes the MRI-certificate if the predefined MRI-requirements are no longer met.

If an electrode has e.g. been replaced during an electrode revision, it is possible that an electrode from another manufacturer is implanted. In this case, the entire system however can no longer be considered safe. Thus, no MRI-certificate should be issued or renewed. In doing so, patient safety is ensured while also alerting the treating physician of the updated MRI-certificate status.

According to a further aspect of the invention, the remote monitoring system upon receipt of the technical and/or medical data sends a second notification to a user prompting the user to take administrative action concerning the MRI-certificate. This procedure incorporates a user, i.e. the treating physician to review the technical and/or medical data in order to decide a suitable action concerning an update of the MRI-certificate status.

According to a further aspect of the invention, the remote monitoring system additionally evaluates data from electronic information systems connected to the remote monitoring system, in particular EHR systems, to assess MRI-compliance of the active implant system.

Evaluating data from further information systems advantageously aids in making a more accurate decision regarding the update of the MRI-certificate status.

According to a further aspect of the invention, the remote monitoring system automatically transmits a change in status of the MRI-certificate to at least one of an MRI-database, an EHR system and to a user and/or a patient via a patient app, push media and/or electronic or postal mail. The change in status of the MRI-certificate is thus communicated in a timely and efficient manner to the user as well as the patient.

According to a further aspect of the invention, the predefined MRI-requirements are that a stimulation amplitude, an electrode impedance and/or an ECG-signal are within a specified range.

The herein described features of the computer-implemented method for performing an administrative action concerning a MRI-certificate of the active implant system are also disclosed for the system for performing an administrative action concerning a MRI-certificate and vice versa.

For a more complete understanding of the present invention and advantages thereof, reference is now made to the following description taken in conjunction with the accompanying drawings. The invention is explained in more detail below using exemplary embodiments, which are specified in the schematic figures of the drawings, in which:

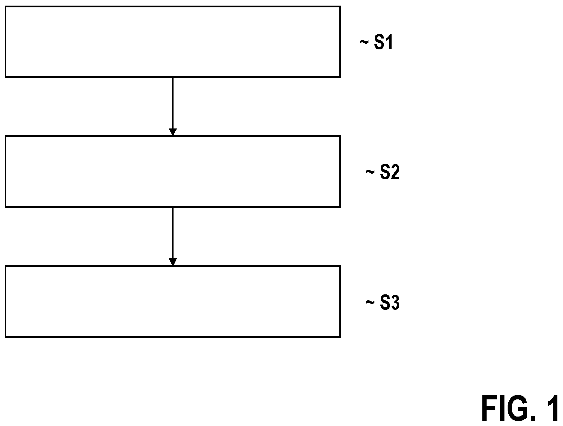

- Fig. 1: shows a flowchart of a computer-implemented method for performing an administrative action concerning a MRI-certificate of the active implant system according to a preferred embodiment of the invention; and
- Fig. 2: shows a diagram of a system for performing an administrative action concerning a MRI-certificate of the active implant system according to the preferred embodiment of the invention.

The computer-implemented method for performing an administrative action 18 concerning an MRI certificate 10 of the active implant system 12 comprises providing S1 the active implant system 12, said active implant system 12 comprising an implantable medical device 12a and electrode leads 12b connected to the implantable medical device 12a.

Furthermore, the method comprises transmitting S2 technical and/or medical data 14 acquired by the active implant system 12, in particular via a relay device 28, to a remote monitoring system 16, based on the technical and/or medical data 14 acquired by the active implant system 12, verifying S3 a MRI-compliance of the active implant system 12, and based on a result of the verification, performing an administrative action 18 concerning the MRI-certificate 10 of the active implant system 12.

The predefined MRI-requirements are that a stimulation amplitude, an electrode impedance and/or an ECG-signal are within a specified range. The technical and/or medical data 14 are also a stimulation amplitude, an electrode impedance and/or an ECG-signal.

Other technical and/or medical data may also be considered to determine MRI suitability of the active implant system 12, wherein any technical and/or medical data that indicates a malfunction of the implantable medical device 12a and/or the electrode leads 12b connected to the implantable medical device 12a may be considered for this purpose.

The result of the verification is thus that the above-mentioned technical and/or medical data are within the specified range or not. If yes, the MRI-certificate 10 is issued or re-issued. If not, the MRI-certificate 10 is revoked.

The method moreover comprises performing the administrative action 18 concerning the MRI-certificate 10 of the active implant system 12 comprising issuing, revoking or re-issuing the MRI-certificate 10 of the active implant system 12 by the remote monitoring system 16.

In addition, the remote monitoring system 16 updates an expiry date of the MRI-certificate 10 in a data storage 20 of the implantable medical device 12a when the MRI-certificate 10 is issued, revoked or re-issued.

The MRI-certificate 10 has a specified period of validity, wherein the active implant system 12 sends a first notification 22 to the remote monitoring system 16 to initiate re-issuing the MRI-certificate 10 at a predefined point in time prior to expiration of the MRI-certificate 10.

Specifically, the active implant system 12 transmits the technical and/or medical data 14, in particular via the relay device 28, to the remote monitoring system 16 required for verifying the MRI-compliance of the active implant system 12 at said predefined point in time prior to expiration of the MRI-certificate 10.

The active implant system 12 sends the technical and/or medical data 14 required for verifying the MRI-compliance of the active implant system 12 with its daily data transmission to the remote monitoring system 16. Subsequently, the remote monitoring system 16 evaluates all daily data transmissions and automatically issues or re-issues the MRI-certificate 10 if the predefined MRI-requirements are met.

By the same token, the remote monitoring system 16 evaluates all daily data transmissions and automatically revokes the MRI-certificate 10 if the predefined MRI-requirements are no longer met.

Alternatively, the remote monitoring system 16 upon receipt of the technical and/or medical data 14 sends a second notification 24 to a user 32 prompting the user 32 to take administrative action 18 concerning the MRI-certificate 10.

Moreover, the remote monitoring system 16 automatically transmits a change in status of the MRI-certificate 10 to at least one of an MRI-database, an EHR system, and to the user 32 and/or a patient via a patient app 30, push media and/or electronic or postal mail.

The remote monitoring system 16 additionally evaluates data from electronic information systems 26 connected to the remote monitoring system 16, in particular EHR systems, to assess the MRI-compliance of the active implant system 12.

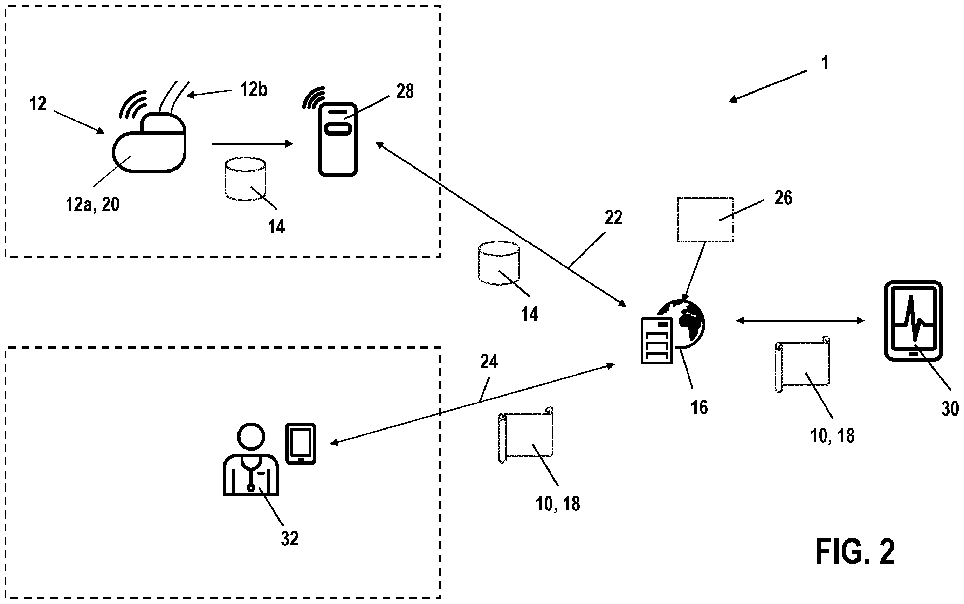

Fig. 2 shows a diagram of a system 1 for performing an administrative action 18 concerning a MRI-certificate 10 of the active implant system 12 according to the preferred embodiment of the invention.

The system 1 comprises an active implant system 12, said active implant system 12 comprising an implantable medical device 12a and electrode leads 12b connected to the implantable medical device 12a and a remote monitoring system 16 configured to exchange data with the active implant system 12.

The active implant system 12 is configured to transmit technical and/or medical data 14 acquired by the active implant system 12, in particular via a relay device 28, to the remote monitoring system 16, wherein the remote monitoring system 16 is further configured to, based on the technical and/or medical data 14 acquired by the active implant system 12, verify a MRI-compliance of the active implant system 12.

Furthermore, based on a result of the verification, the remote monitoring system 16 is configured to perform an administrative action 18 concerning the MRI-certificate 10 of the active implant system 12.

Although specific embodiments have been illustrated and described herein, it will be understood by those skilled in the art that a variety of alternative and/or equivalent implementations exist. It should be noted that the exemplary embodiment or exemplary embodiments are examples only and are not intended to limit the scope, applicability or configuration in any way.

Rather, the foregoing detailed description provides the skilled person with a convenient guide to implementing at least one exemplary embodiment, it being understood that various changes in the scope of functionality and arrangement of the elements may be made without departing from the scope of the appended claims and their legal equivalents.

In general, this application intends to cover modifications or adaptations or variations of the embodiments disclosed herein. For example, a sequence of method steps may be modified. The method may further be carried out sequentially or in parallel, at least in part.

### Reference Signs

- 1: system
- 10: MRI-certificate
- 12: active implant system
- 12a: implantable medical device
- 12b: electrode leads
- 14: technical and/or medical data
- 16: remote monitoring system
- 18: administrative action
- 20: data storage
- 22: first notification
- 24: second notification
- 26: electronic information systems
- 28: relay device
- 30: patient-app
- 32: user
- S1-S3: method steps

## Claims

1. Computer-implemented method for performing an administrative action (18) concerning an MRI-certificate (10) of the active implant system (12), comprising the steps of:
providing (S1) the active implant system (12), said active implant system (12) comprising an implantable medical device (12a) and electrode leads (12b) connected to the implantable medical device (12a);
transmitting (S2) technical and/or medical data (14) acquired by the active implant system (12), in particular via a relay device (28), to a remote monitoring system (16);
based on the technical and/or medical data (14) acquired by the active implant system (12), verifying (S3) a MRI-compliance of the active implant system (12); and
based on a result of the verification, performing an administrative action (18) concerning the MRI-certificate (10) of the active implant system (12).

2. Computer-implemented method of claim 1, wherein performing the administrative action (18) concerning the MRI-certificate (10) of the active implant system (12) comprises issuing, revoking or re-issuing the MRI-certificate (10) of the active implant system (12) by the remote monitoring system (16).

3. Computer-implemented method of claim 2, wherein the remote monitoring system (16) updates an expiry date of the MRI-certificate (10) in a data storage (20) of the implantable medical device (12a) when the MRI-certificate (10) is issued, revoked or re-issued.

4. Computer-implemented method of any one of the preceding claims, wherein the MRI-certificate (10) has a specified period of validity, wherein the active implant system (12) sends a first notification (22) to the remote monitoring system (16) to initiate re-issuing the MRI-certificate (10) at a predefined point in time prior to expiration of the MRI-certificate (10).

5. Computer-implemented method of claim 4, wherein the active implant system (12) transmits the technical and/or medical data (14) to the remote monitoring system (16) required for verifying the MRI-compliance of the active implant system (12) at said predefined point in time prior to expiration of the MRI-certificate (10).

6. Computer-implemented method of claim 5, wherein the active implant system (12) sends the technical and/or medical data (14) required for verifying the MRI-compliance of the active implant system (12) with its daily data transmission to the remote monitoring system (16).

7. Computer-implemented method of claim 6, wherein the remote monitoring system (16) evaluates all daily data transmissions and automatically issues or re-issues the MRI-certificate (10) if the predefined MRI-requirements are met.

8. Computer-implemented method of claim 6 or 7, wherein the remote monitoring system (16) evaluates all daily data transmissions and automatically revokes the MRI-certificate (10) if the predefined MRI-requirements are no longer met.

9. Computer-implemented method of any one of the preceding claims, wherein the remote monitoring system (16) upon receipt of the technical and/or medical data (14) sends a second notification (24) to a user 32 prompting the user 32 to take administrative action (18) concerning the MRI-certificate (10).

10. Computer-implemented method of claim 9, wherein the remote monitoring system (16) automatically transmits a change in status of the MRI-certificate (10) to at least one of an MRI-database, an EHR system, and to the user 32 and/or a patient via a patient app (30), push media and/or electronic or postal mail.

11. Computer-implemented method of any one of the preceding claims, wherein the remote monitoring system (16) additionally evaluates data from electronic information systems (26) connected to the remote monitoring system (16), in particular EHR systems, to assess the MRI-compliance of the active implant system (12).

12. Computer-implemented method of any one of the preceding claims, wherein the predefined MRI-requirements are that a stimulation amplitude, an electrode impedance and/or an ECG-signal are within a specified range.

13. System (1) for performing an administrative action (18) concerning an MRI-certificate (10) of the active implant system (12), comprising:
an active implant system (12), said active implant system (12) comprising an implantable medical device (12a) and electrode leads (12b) connected to the implantable medical device (12a); and a remote monitoring system (16) configured to exchange data with the active implant system (12), wherein the active implant system (12) is configured to transmit technical and/or medical data (14) acquired by the active implant system (12), in particular via a relay device (28), to the remote monitoring system (16), wherein the remote monitoring system (16) is further configured to, based on the technical and/or medical data (14) acquired by the active implant system (12), verify a MRI-compliance of the active implant system (12), and based on a result of the verification, the remote monitoring system (16) is configured to perform an administrative action (18) concerning the MRI-certificate (10) of the active implant system (12).

14. Computer program with program code to perform the method of any one of claims 1 to 12 when the computer program is executed on a computer.

15. Computer-readable data carrier containing program code of a computer program for performing the method of any one of claims 1 to 12 when the computer program is executed on a computer.
